# EUROPEAN PATENT APPLICATION

(11) **EP 4 026 477 A1**
(43) Date of publication of application: **13.07.2022**
(21) Application number: 21184996.3
(22) Date of filing: 12.07.2021
(51) Int. Cl.: A61B 1/00, A61B 1/005, A61B 1/018, A61B 1/05, A61B 1/06, A61B 1/303, A61B 17/42, A61B 17/44, A61B 1/008

(54) **FOLDABLE AND ADJUSTABLE ENDOSCOPIC INSTRUMENT TO ATTACH THE FETUS SHOULDER**

(30) Priority: 12.01.2021 IT 202100000287
(71) Applicant: H-Opera S.r.l., 84084 Fisciano (SA) (IT)
(72) Inventor: CATALDO, Emilio, 84091 BATTIPAGLIA (SA) (IT); GIUGLIANO, Luigi, 84125 SALERNO (IT); RUGGIERO, Alessandro, 84125 SALERNO (IT); CICINELLI, Ettore, 70054 GIOVINAZZO (BA) (IT)
(74) Representative: Conversano, Gabriele

(57) **Abstract**

Flexible and adjustable endoscope configured to grasp the fetus' shoulder during a delivery final step, comprising: an outer handle (6), a hollow tube (1) provided with a flexible end (2), configured to take a first substantially rectilinear configuration and a second hook configuration, characterized in that said flexible end is configured to be introduced in vagina in rectilinear configuration and to change shape, thus taking a hook configuration, after passing beyond the section where the fetus head and shoulders are, and in that the configuration of said flexible end (1) can be controlled by a kinematism whose controls are positioned inside said handle (6).

## Description

Dystocia is defined as a cephalic vaginal delivery, where, after failure of the traction exerted on the fetal head, it is needed to carry out further obstetric maneuvers to favour the shoulder releasing and the baby delivery.

Shoulder dystocia occurs when the baby's anterior, or less commonly, the back shoulder, is caught on the maternal pubic symphysis or the sacral promontory. Both shoulders are exceptionally caught at the pelvis.

The incidence of the dystocia phenomenon is very variable and is estimated between 0.58% and 0.70% of all deliveries. Fetal macrosomia and diabetes are considered risk factors for shoulder dystocia. Anyway, it is to be highlighted that the most part of dystocias occurs in women not suffering from risk factors and so, dystocia has to be considered unpredictable. For these reasons, scientific companies do not recommend elective labor induction or Caesarian section for all the women with probable fetal macrosomia and/or diabetes. Specifically, Caesarian section should be programmed in women with suspected macrosomia with an estimation of fetal weight higher than 5000 g if not suffering from diabetes, and higher than 4500 g if suffering from diabetes.

The phenomenon of dystocia, caused by the difficulty to extract the baby during delivery, has different consequences for the baby.

The diagnosis of incipient dystocia can be done in cases of difficulty of face and chin releasing, in case the head of the baby remains strictly applied at the vulva (or goes back), in case of not presentation of the head of the baby, or of not descent of the shoulders.

Literature data have highlighted that if dystocia is not resolved, 47% of the babies dies in 5 minutes after the head expulsion.

A significant mortality and morbidity can occur also when dystocia is faced appropriately; increases of the incidence of postpartum bleedings as well as of third-degree lacerations are reported in fact, irrespective of the number and kind of maneuvers required to carry out the delivery.

In case of dystocia, it is essential to face the problem rapidly and with skill to avoid dangerous phenomena as acute hypoxia, mother and neonatal trauma.

In case of dystocia in fact the death of the baby is caused by the pressure gradient occurring between the expulsed head and the body retained; hence the venous return from the encephalic circle to the heart is hindered, while arterial blood keeps flowing to the head; this leads to asystole, hypovolemic shock and sudden death. The perinatal death is between 16% and 29%.

From the clinical evidence, it results that the main pathogenetic factor is the vascular trauma with the sudden and radical alteration of the return blood flows to the heart.

In case of a dystocia, in addition to a series of propaedeutic operations aiming at facing the problem at best, it is needed to carry out a series of maneuvers to free the baby.

The most known obstetric maneuvers for dystocia, with increasing invasivity, are:
- McRoberts maneuver (forced ventral hyperflexion of the mother's legs to her admonen);
- Rubin maneuver (a suprapubic pressure is exerted on the mother's abdomen to push the baby's anterior shoulder in ventral direction on the pelvis oblique diameter);
- Mazzanti maneuver (a suprapubic pressure is carried out with the hand palm centrally in antero-posterior direction on the anterior shoulder);
- Rubin 2 maneuver (the (dorsal) posterior face of the anterior shoulder is pushed towards the fetal thorax to bring the bisacromial diameter from antero-posterior to oblique);
- Jacquimier maneuver (the posterior shoulder is reached while the other hand pushes the anterior shoulder on pubis; by reaching with the hand the posterior arm of the fetus in vagina, he is rotated on the ventral plane and he is extracted from the vagina);
- Zavanelli maneuver (if the previous maneuvers fail, the fetal head will be pushed again in the birth canal and a caesarian section is carried out) .

The obstetric maneuvers aimed at resolving dystocia are associated to a high mother morbidity even if caried out correctly. In fact, soft tissues lesions are frequent (III and IV grade vagino-perineal lacerations, postpartum uterine atony and consequent postpartum bleedings - with incidence about 11% - uterine rupture, IV grade postpartum pelvis infections - with incidence about 3.8% - pubic symphysis diastasis and maternal neuropathies).

The main complications are brachial plexus lesions, in between 4 and 16% of cases. They represent the most frequent reason of medico-legal matters relating to dystocia. The risk of brachial plexus lesions correlated to dystocia often seems independent of the operator skills and experience. Erb's palsy is the most frequent pathological consequence of a dystocia. It occurs with a shoulder inner rotation and adduction, elbow extension and pronation and little wrist extension. It is caused by damage to the nerve roots C5-C6. Klumpke paralysis, less frequent than the previous one, is caused by damage to the nerve roots C8-T1. It determines weakening of hand muscles and of flexion and extension of the hand fingers, with claw behaviour; the hand cannot be completely closed in a fist.

Other complications are clavicle and humerus fractures, less severe from a functional recovery point of view, even if cases of lesions to the lungs and vascular structures therebelow have been reported. Shoulder dystocia can rarely cause a lesion to the cervical column with baby's death.

There exists scientific evidence to show that the mother pushing can cause these lesions irrespective of the outer maneuvers.

It is clear, from what described, that the dystocia phenomenon represents a real problem in medical and surgery field, considering, among other things, many severe consequences for the baby and the mother, which often can lead to death.

### Aim of the invention

Aim of the present invention is to provide a tool configured to facilitate the solution of the problem of dystocia, thus allowing the execution of an aid maneuver for the baby expulsion.

### Description of the invention

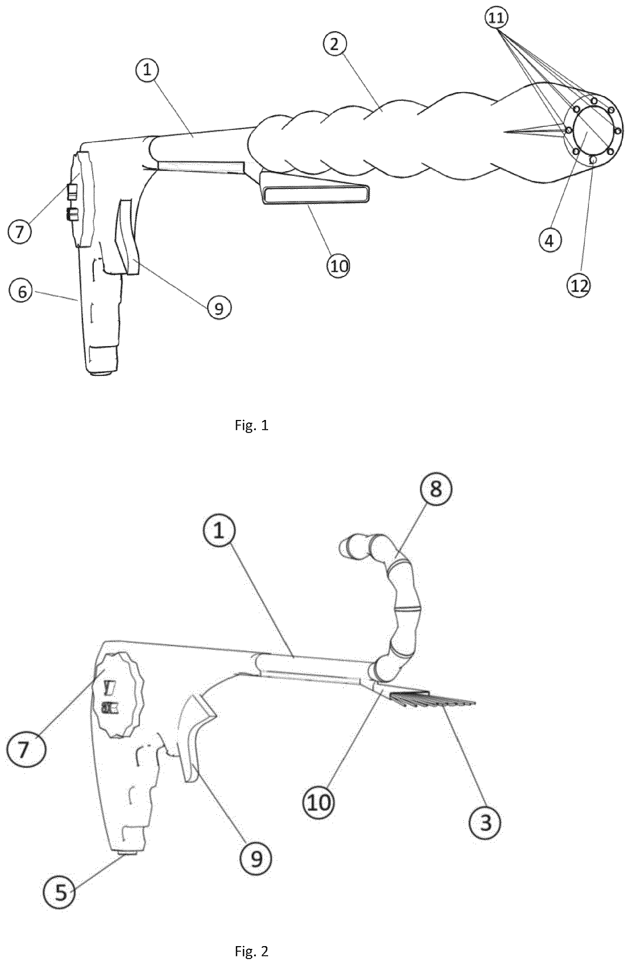

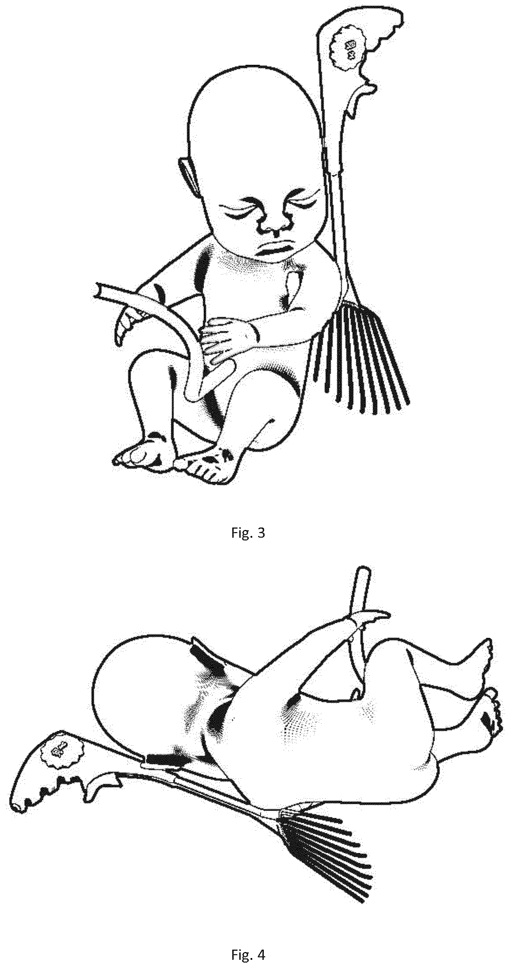

As it is shown in the appended figures, object of the present invention is a flexible and adjustable endoscope to grasp the fetus' shoulder comprising an outer handle (6), a hollow tube (1) provided with a flexible end (2), configured to take a first straight (or substantially rectilinear) configuration, as it is shown in figure 1, and a second curved configuration, shaped as a hook (8) with a variable curvature radius.

The device is realized so that the configuration of said flexible end (1) can be controlled by a kinematism whose controls are positioned inside said outer handle (6).

In particular, through the controls provided on said device, the curvature radius of said flexible end (2), when in configuration of hook (8), can be varied on two planes orthogonal to each other, thus allowing the operator to grasp the fetus' shoulder and to carry out an optimal maneuver for the dystocia solution.

It is clear that such possibility is offered by the provision of a flexible end (2) configured to be introduced in vagina (and possibly in uterine cervix) and to change shape, thus taking a hook configuration, after passing beyond the section where the fetus head and shoulders are.

Said tube (1) has preferably a diameter about 15 mm, and is configured to house therein the control means of said flexible end, which in a preferred embodiment comprise a plurality of cables.

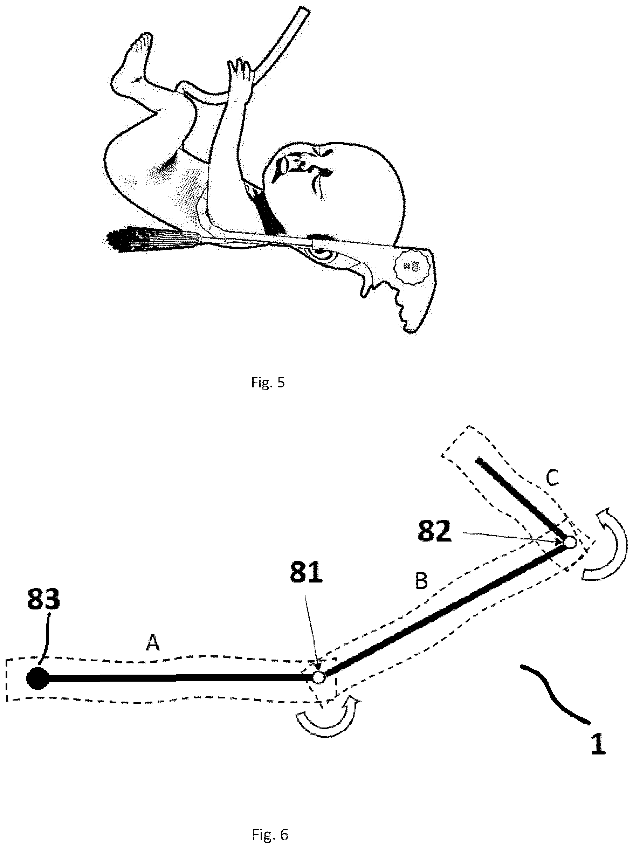

In another embodiment shown in figure 6, the tube (1) comprises therein a plurality of rectilinear rigid portions (A, B, C), hinged to each other, and a plurality of electric actuation means (81, 82) positioned at each one of the hinges fastening said rectilinear rigid portions. So, the tube can pass to the rectilinear configuration to the hook configuration by sequential actuation of said actuation means (81, 82).

Moreover, the device comprises preferably a further electric actuation means (83), schematically shown in figure 6, configured to make said tube (1) rotate on its own axis. In this way, when the tube is in rectilinear configuration the space plane can be determined in which the same will be arranged after taking its hook configuration.

Moreover, all the used actuation means (81, 82, 83) comprise preferably irreversible type mechanisms. In other words, if subjected to a force exerted from outside on the tube, the actuation means do not rotate, thus allowing the tube to keep its own configuration rigidly.

It is clear that inside the tube there are provided connecting means of said electric actuation means, which allow the control by means of controls provided in said outer handle.

Inside the handle, control electronic means can be provided, configured to control said actuation means so that they carry out a sequence of predetermined movements.

In this way, the operator cannot carry out sequences of movements with the tube (1), to pass from the rectilinear configuration to the hook one, which could be dangerous.

Moreover, the device comprises preferably, parallel to said tube (1) and fastened thereto, a further operation channel inside which a fan-shaped laparoscopic retractor (3) is housed, configured to push the fetal bisacromial diameter along the oblique diameter of the mother pelvis, without hooking it, if this latter operation results difficult to be carried out.

To such aim, the device comprises also control means (9) of said laparoscopic retractor (3), preferably trigger shaped, provided on said outer handle (6).

In a preferred embodiment, said flexible end is configured to take the shape of a hook with a curvature radius about 3 cm, so that the fetus axillary area can be grasped easily.

In a preferred embodiment the device comprises also a camera (4) and a light source (11), fastened at the end of said flexible end and controllable by means of suitable control means positioned on said outer handle (6).

The provision of the camera, which can be connected by means of any technology known per se to an outer monitor, allows the operator to carry out a correct positioning of the tool to improve the fetus grasping.

Using the flexible and adjustable endoscope according to the present invention allows to visualize, and possibly to anchor, structures which otherwise would be difficult to be identified and reached by the obstetrician.

Said outer handle (6) comprises preferably all the electronic components needed for a wireless transmission of the data acquired by the camera to a post processing system in real time, able to allow a fluid visualization and in real time, on an outer monitor, of images assisting the operator while carrying out the dystocia solution maneuvers. The flexible and adjustable endoscope to grasp the fetus' shoulder can be also used in the expulsive step of the delivery to visualize possible obstacles (prolapse or umbilical cord prolapse etc.) and to estimate visually the cervix, fetus or amniotic liquid conditions during the labor active step.

Moreover, the device comprises also, integrated inside said flexible end and said hollow tube, a channel for water or saline solution injection. To such aim, the outer handle (6) is provided with fastening means (5) to which a water or saline solution supply, for example vials or glass bottles of the type commonly used for drips, can be coupled.

Said handle can also contain inside a peristaltic pump configured to push said saline solution towards expulsion openings, positioned at the end of said flexible end and configured to supply the saline solution so that both the operating field and the camera optics is cleaned. In such a way, it is possible to obtain the most possible clear and not obstructed by bloody effusions view of the tract under surgery.

## Claims

1. Flexible and adjustable endoscope configured to grasp the fetus' shoulder during a delivery final step, comprising:
- an outer handle (6),
- a hollow tube (1) provided with a flexible end (2), configured to take a first substantially rectilinear configuration and a second hook-shaped configuration,
**characterized in that**
said flexible end is configured to be introduced in vagina in rectilinear configuration and to change shape, thus taking a hook configuration, after passing beyond the section where the fetus head and shoulders are,
and **in that**
the configuration of said flexible end (1) can be controlled by a kinematism whose controls are positioned inside said handle (6).

2. Tool according to claim 1, **characterized in that** said the tube (1) comprises therein a plurality of rectilinear rigid portions (A, B, C), hinged to each other, and a plurality of electric actuation means (81, 82) positioned at each one of the hinges fastening said rectilinear rigid portions.

3. Tool according to claim 2, **characterized in that** said electric actuation means comprise an irreversible type mechanism.

4. Tool according to claim 2, comprising further another electric actuation means (83) configured to make said tube (1) rotate on its own axis.

5. Tool according to any one of the preceding claims, **characterized in that** said hook has a variable curvature radius along its own axis.

6. Tool according to any one of the preceding claims, **characterized in that** the curvature radius of said hook can be varied on two planes orthogonal to each other.

7. Tool according to any one of the preceding claims, **characterized in that** said tube (1) has a diameter about 15 mm, and is configured to house therein the control means of said flexible end.

8. Tool according to any one of the preceding claims, **characterized in that** it comprises further, parallel to said tube (1) and fastened thereto, a further operation channel inside which a fan-shaped laparoscopic retractor (3) is housed, configured to push the fetal bisacromial diameter along the oblique diameter of the mother pelvis, without hooking it, and control means (9) of said laparoscopic retractor (3).

9. Tool according to any one of the preceding claims, **characterized in that** said flexible end is configured to take the shape of a hook with a curvature radius about 3 cm, so that the fetus axillary area can be grasped easily.

10. Tool according to any one of the preceding claims, **characterized in that** it comprises also a camera (4) and a light source (11), fastened at the end of said flexible end and controllable by means of suitable control means positioned on said outer handle (6).

11. Tool according to any one of the preceding claims, **characterized in that** it comprises also, integrated inside said flexible end and said hollow tube, a channel for water or saline solution injection.

12. Tool according to claim 8, **characterized in that** said outer handle (6) is provided with fastening means (5) to which a water or saline solution supply can be coupled.

13. Tool according to claim 9, **characterized in that** said handle (6) comprises a peristaltic pump configured to push said saline solution towards expulsion openings, positioned at the end of said flexible end and configured to supply the saline solution so that both the operating field and the camera optics is cleaned.
